# FASCICULE DE BREVET EUROPEEN

(11) **EP 0 970 691 B1**
(45) Date de publication et mention de la délivrance du brevet: **16.07.2003**
(21) Numéro de dépôt: 99401522.0
(22) Date de dépôt: 18.06.1999
(51) Int. Cl.: A61K 7/48

(54) **Produit pour application topique contenant une lipase, un précurseur de vitamine et un alcool gras**
Produkt zur topischen Anwendung, das eine Lipase, einen Vitaminvorläufer und einen Fettalkohol enthaelt
Composition for topical application containing a lipase, vitamin precursor and a fatty alcohol

(30) Priorité: 06.07.1998 FR 9808615
(43) Date de publication de la demande: 12.01.2000
(73) Titulaire: L'OREAL, 75008 Paris (FR)
(72) Inventeur: Boussouira, Boudiaf, 75020 Paris (FR); Pham, Dang-Man, 94370 Sucy en Brie (FR)
(74) Mandataire: Renard, Emmanuelle

(56) Documents cités:
- EP-A- 0 487 404
- EP-A- 0 710 478
- FR-A- 2 101 044
- PATENT ABSTRACTS OF JAPAN vol. 015, no. 297 (C-0854), 29 juillet 1991 (1991-07-29) & JP 03 109311 A (PENTEL KK), 9 mai 1991 (1991-05-09)

## Description

La présente invention a pour objet un produit pour application topique apte à libérer sur la peau un actif cosmétique et/ou dermatologique, et son utilisation ' pour le traitement cosmétique de la peau, y compris le cuir chevelu.

Il est connu d'introduire dans les compositions cosmétiques et/ou dermatologiques des actifs en vue d'apporter des traitements spécifiques à la peau, par exemple pour lutter contre le dessèchement, le vieillissement ou la pigmentation de la peau, pour traiter l'acné ou certaines maladies de la peau (eczéma, psoriasis), pour combattre les surcharges pondérales, ou pour favoriser la restructuration de la peau ou son renouvellement cellulaire.

Par exemple, l'acide ascorbique (ou vitamine C) est connu pour stimuler la croissance du tissu conjonctif, et notamment celle du collagène. Il permet aussi de renforcer les défenses du tissu cutané contre les agressions extérieures telles que le rayonnement ultraviolet ou la pollution. Il est également utilisé pour enlever les taches et la pigmentation de la peau, et aussi pour favoriser la cicatrisation de la peau.

Il est connu aussi que l'application de rétinol ou vitamine A permet de lutter notamment contre le vieillissement cutané et contre certains désordres de la peau tels que l'acné ou les troubles de la kératinisation ou de la cicatrisation.

En outre, les tocophérols, tels que la vitamine E, sont connus pour posséder des propriétés antioxydantes vis à vis des phospholipides de la membrane cellulaire et des propriétés antiradicalaires (voir "Radicaux libres et Vitamine E" de J.B. Chazan et M. Szulc - Cah. Nutr. Diet. 1987, 6, XXII, 1, pages 66 à 76).

Malheureusement, certains actifs, et en particulier ceux cités ci-dessus, sont instables et sont sensibles à des facteurs extérieurs tels que la lumière ou la chaleur. Cette instabilité va à l'encontre de l'efficacité recherchée et peut, de plus, être source de désagréments pour l'utilisateur, par exemple lorsque l'instabilité de l'actif entraîne des modifications de couleur et/ou d'odeur de la composition le contenant.

Aussi, différents moyens ont été envisagés pour stabiliser ces actifs. Un de ces moyens consiste par exemple à bloquer le site réactif de l'actif par estérification avec notamment des dérivés phosphatés, sulfatés ou alkylés et à utiliser ces dérivés à la place de l'actif libre. Malheureusement, ces dérivés présentent une efficacité moins bonne que l'actif libre.

Il a été aussi envisagé d'utiliser des précurseurs de tels actifs, qui, après application sur la peau, sont coupés par les enzymes cutanés et libèrent alors l'actif libre. Ainsi, le document EP-A-487404 divulgue l'utilisation d'un dérivé glucosylé de l'acide ascorbique, dans des compositions dermatologiques, facilement hydrolysé par les enzymes cutanés et donc apte à libérer de l'acide ascorbique lorsque ces compositions sont appliquées sur la peau. Mais l'utilisation de tels dérivés ne permet pas une libération rapide et en quantité suffisante d'acide ascorbique à la surface de la peau.

Il subsiste donc le besoin d'un produit pour application topique contenant des vitamines utilisées en cosmétique et/ou dermatologie, dans lequel ces dernières conservent toutes leurs propriétés et donc leur efficacité au cours du temps.

La demanderesse a ainsi décrit dans le brevet EP 710478 que l'utilisation d'une enzyme particulier, la lipase, associé à des esters de vitamines instables, comme la vitamine A (rétinol) ou la vitamine C (acide ascorbique) permettait d'éviter les inconvénients de l'art antérieur.

Cependant, la Demanderesse vient de découvrir que la libération rapide et en quantité suffisante de vitamines peut être améliorée en introduisant des alcools de C6 à C22 dans la composition.

Aussi, la présente invention a pour objet un produit pour application topique, caractérisé en ce qu'il comprend au moins une enzyme qui est une lipase, au moins un précurseur d'une vitamine utilisée en cosmétique et/ou dermatologie qui est un ester comportant au moins une fonction ester à chaîne linéaire ou ramifiée, saturée ou insaturée, ayant de 2 à 25 atomes de carbone et au moins un alcool de C6 à C22, le rapport pondéral entre ledit alcool et ledit précurseur étant de 5 à 15/1.

Par produit, on entend aussi bien la partie cosmétique ou dermatologique (ensemble de la composition) que le dispositif commercial pouvant comporter un récipient dans lequel sont logés l'enzyme et le précurseur isolés l'un de l'autre.

La lipase est une enzyme connue pour hydrolyser les triglycérides en mono- et diglycérides, en glycérol et en acides gras libres. Elle est en particulier utilisée dans les détergents (voir l'article "Lipases as detergent components", H. Andree et al., Journal of Applied Biochemistry, 1980, vol. 2, pages 218 à 229) afin de permettre l'élimination des taches graisseuses telles que celles provenant des matières grasses de friture, d'huiles, de sebum ou de cosmétiques gras comme les rouges à lèvres. Du fait de sa propriété dé couper les triglycérides, elle a été utilisée dans le domaine cosmétique sous forme immobilisée pour nettoyer la peau (voir par exemple US-A-4556554).

La lipase selon l'invention doit être suffisamment stable pour conserver son activité enzymatique. Elle appartient au groupe des enzymes de classification EC 3.1.1.3., ce qui correspond à une lipase qui coupe les liaisons ester en positions 1 et 3 d'un triglycéride. Elle peut être choisie par exemple parmi celles vendues sous les dénominations commerciales "lipase SP644" et "lipolase 100 L" par la société Novo Nordisk.

La lipase peut être utilisée dans le produit selon l'invention en une quantité allant de 0,05 % à 30 % en poids, de préférence de 0,1 à 10 % en poids par rapport au poids total de la composition, et mieux de 0,1 à 5 % en poids par rapport au poids total de la composition.

Les vitamines auxquelles s'applique l'invention sont celles comportant au moins une fonction hydroxyle, et notamment les vitamines estérifiables telles que le rétinol (vitamine A) et ses dérivés, le tocophérol ou ses dérivés, l'acide ascorbique (vitamine C) et ses dérivés.

L'ester utilisé selon l'invention est un ester comportant une ou plusieurs fonctions ester à chaîne linéaire ou ramifiée, saturée ou insaturée, ayant de 2 à 25 atomes de carbone, comportant éventuellement un ou plusieurs substituants. La chaîne de la fonction ester est choisie en particulier parmi les radicaux acyle, benzoyle, alkylbenzoyle, acylbenzoyle, 2-hydroxyphénylacétyle, éventuellement substitués. Le substituant peut être en particulier un radical hydroxylé.

Selon une réalisation préférée de l'invention, la chaîne de la fonction ester a de 12 à 18 atomes de carbone.

Il s'agit par exemple d'un ester choisi parmi les esters d'acide laurique, d'acide palmitique, d'acide stéarique, d'acide cétylique, d'acide myristique, d'acide linoléique, d'acide octanoïque, d'acide oléique, ou également d'esters d'acide butyrique, d'acide propionique, d'acide acétique, ou aussi des esters d'acide hydroxylé tel que l'acide salicylique ou l'acide lactique, ou des mélanges de ces esters.

L'ester utilisé selon l'invention peut être par exemple le mono- et dilaurate de dihydroxyacétone, le mono- et distéarate de dihydroxyacétone, le mono- et dipalmitate de dihydroxyacétone, le palmitate d'ascorbyle, le laurate d'ascorbyle, le myristate d'ascorbyle, le stéarate d'ascorbyle, le nicotinate d'ascorbyle, l'acétate de tocophérol, le linoléate de tocophérol, le palmitate de rétinyle, le propionate de rétinyle, l'acétate de rétinyle, le butyrate de rétinyle, l'octanoate de rétinyle, le laurate de rétinyle, l'oléate de rétinyle, le linoléate de rétinyle.

L'ester est utilisé dans le produit selon l'invention en une quantité allant de 0,1 à 50 % en poids, et de préférence de 0,5 à 10 % en poids par rapport au poids total de la composition quand il s'agit d'ester de vitamine, et de 5 à 30 % en poids par rapport au poids total de la composition quand il s'agit d'ester de cétones et notamment d'ester de dihydroxyacétone.

Les alcools de C6 à C22 utilisés selon l'invention sont de préférence des alcools dont la chaîne carbonée est, saturée ou insaturée, linéaire, branché ou cyclique. Ainsi, on peut notamment citer l'alcool cétylique, stéarylique, isostéarylique, cétylstéarylique, le béhénylstéaryloctyldodécanol, le menthol ou le cholestérol.

Avantageusement, les alcools de C6 à C22, préférentiellement de C8 à C22, utilisés sont ceux dont la chaîne carbonée est, saturée ou insaturée, linéaire ou branché. On peut ainsi plus particulièrement citer l'alcool cétylique, stéarylique, isostéarylique, cétylstéarylique et le béhénylstéaryloctyldodécanol.

Le produit selon l'invention est préférentiellement dépourvu d'acides de C6 à C22 et d'esters d'acides de C6 à C22, tels que l'acide stéarique, les mono, di ou triesters de C6 à C22 de glycérol.

Selon une première variante de l'invention, on introduit la lipase, le précurseur et les alcools de C6 à C22 utilisés selon l'invention dans une composition unique, qui est, de préférence, préparée juste avant l'emploi.

Selon une seconde variante, la lipase et le précurseur sont conditionnés de sorte à ne pas être en contact l'un avec l'autre, par exemple dans deux compositions différentes, qui peuvent être soit mélangées au moment de l'application soit appliquées de façon successive ou décalée dans le temps.

Selon cette seconde variante, les alcools de C6 à C22 utilisés selon l'invention peuvent être compris dans l'une ou l'autre des compositions, et éventuellement dans les deux compositions.

On peut par exemple disposer les compositions dans deux compartiments, qui sont en communication avec un conduit commun, d'où elles peuvent sortir en se mélangeant avant application sur la peau. De tels dispositifs de conditionnement en deux compartiments sont par exemple décrits dans les documents FR-A-2045559, FR-A-2105332, FR-A-2258319, FR-A-2293375, FR-A-2586913 ou FR-A-2643615.

On peut aussi réaliser l'une des compositions sous forme encapsulée et/ou sous forme de microcapsules ou de microgranulés immergés dans l'autre composition, les microcapsules ou les microgranulés étant écrasés au moment de l'application par frottement sur la peau, ce qui permet ainsi le mélange de la lipase, du précurseur et des alcools de C6 à C22 et la libération de la vitamine libre sur la peau.

Le produit selon l'invention comporte avantageusement un milieu approprié pour une application topique, destiné aux domaines cosmétique et/ou dermatologique.

Aussi, l'invention a en outre pour objet l'utilisation du produit tel que défini ci-dessus pour préparer une pommade ou un onguent dermatologique destiné au traitement thérapeutique de la peau.

L'invention a encore pour objet l'utilisation du produit tel que défini ci-dessus pour le traitement cosmétique de la peau.

Le milieu cosmétiquement et/ou dermatologiquement acceptable comprend généralement de l'eau, ou un mélange d'eau et de corps gras, ou un mélange de corps gras.

Comme corps gras utilisables dans l'invention, on peut citer les huiles minérales (vaseline, huile minérale), les huiles végétales et leurs dérivés hydrogénés, les huiles animales, les huiles de synthèse, les huiles siliconées (diméthicone, cyclométhicone) et les huiles fluorées. Comme autres corps gras, on peut encore citer les acides gras et les cires.

En particulier, le produit peut se présenter sous forme de solutions aqueuses, alcooliques ou hydroalcooliques, de gels hydrophiles ou lipophiles, de microémulsions, d'émulsions eau-dans-huile ou huile-dans-eau ou eau-dans-huile-dans-eau ou huile-dans-eau-dans-huile ayant l'aspect d'une crème ou d'un gel, éventuellement aptes à mousser, sous forme d'aérosol, ou encore sous forme de dispersions vésiculaires contenant des lipides ioniques et/ou non ioniques. Ces formes galéniques sont préparées selon les méthodes usuelles des domaines considérés.

De façon connue, le milieu approprié pour une application topique selon l'invention peut contenir également des adjuvants habituels dans le domaine cosmétique ou dermatologique, tels que les gélifiants hydrophiles ou lipophiles, les tensioactifs, les actifs hydrophiles ou lipophiles, les conservateurs, les antioxydants, les solvants, les parfums, les charges, les filtres et les matières colorantes.

Les quantités des différents constituants du produit selon l'invention sont celles classiquement utilisées dans les domaines considérés.

Bien, l'homme du métier veillera à introduire dans ce produit des composés de nature et en quantité ne venant pas interférer avec les objectifs de la présente invention.

Le produit selon l'invention peut constituer notamment des produits de protection, de traitement ou de soin pour le visage, pour le cou, pour les mains ou pour le corps, des produits de bronzage artificiel ou des produits pour les cheveux, et notamment pour le soin du cuir chevelu, par exemple sous forme de shampooings, de lotions traitantes, de crèmes ou de gels coiffants, de lotions ou de gels antichute.

Les exemples qui suivent sont donnés à titre illustratif afin de mieux faire comprendre l'invention. Les quantités indiquées sont des pourcentages en poids, sauf mention contraire.

### EXEMPLES

### Exemple 1

### Influence d'alcools sur l'hydrolyse du rétinyl palmitate à 0,1% en présence de 0,1% de lipase

Différents alcools sont testés à 1% avec du rétinyl palmitate à 0,1% en présence de 0,1% de lipase dans un mileu : Heptane/H2O 40/60%, CaCl2 50mM, 16 heures de contact.

Les résultats concernant les pourcentages d'hydrolyse du rétinyl palmitate sont rassemblés dans le tableau 1 suivant.

**Tableau 1**

| Composés testés | % d'hydrolyse |
|---|---|
| témoin | 23 |
| alcool stéarylique | 94 |
| alcool isostéarylique | 95 |
| alcool cétylique | 94 |
| alcool cétylstéarylique | 94 |
| menthol | 48 |
| cholestérol | 30 |
| béhénylstérayloctyldodécanol | 93 |

On constate ainsi que le taux d'hydrolyse du rétinyl palmitate est fortement augmenté en présence des alcools tels que définis dans la présente invention.

### Exemple 2

### Influence sur l'hydrolyse du rétinyl palmitate à 0,1% en présence de 0,1% de lipase de différents taux d'alcool isostéarylique

Différents taux d'alcool isostéarylique sont testés avec du rétinyl palmitate à 0,1% en présence de 0,1% de lipase dans un mileu : Heptane/H2O 40/60%, CaCl2 50mM, après 90 minutes de réaction.

Les résultats concernant les pourcentages d'hydrolyse du rétinyl palmitate sont rassemblés dans le tableau 2 suivant.

**Tableau 2**

| % d'alcool isostéarylique | % d'hydrolyse |
|---|---|
| 0 | 25% |
| 0,1 % | 68% |
| 0,25% | 80% |
| 0,5% | 88% |
| 1% | 90% |
| 2% | 93% |
| 3% | 69% |
| 4% | 56% |
| 6% | 30% |

On constate sur ce tableau que ce taux d'hydrolyse est très élevé dans les conditions de la présente invention à savoir lorsque le rapport pondéral entre ledit alcool et ledit précurseur étant de 0,25 à 30/1.

### Exemple 3

### Influence sur l'hydrolyse du rétinyl palmitate à 0,1% en présence de 0,1% de lipase de différents taux d'alcool stéarylique

Différents taux d'alcool stéarylique sont testés avec du rétinyl palmitate à 0,1% en présence de 0,1% de lipase dans un mileu : Heptane/H2O 40/60%, CaCl2 50mM, après 16 heures de réaction.

Les résultats concernant les pourcentages d'hydrolyse du rétinyl palmitate sont rassemblés dans le tableau 3 suivant.

**Tableau 3**

| % d'alcool stéarylique | % d'hydrolyse |
|---|---|
| 0% | 24% |
| 0,025% | 41% |
| 0,1% | 73% |
| 0,2% | 84% |
| 0,25% | 86% |
| 0,5% | 91% |
| 1% | 95% |

On constate sur ce tableau que ce taux d'hydrolyse est très élevé dans les conditions de la présente invention à savoir lorsque le rapport pondéral entre ledit alcool et ledit précurseur étant de 0,25 à 30/1.

### Exemple 4 : Crème de soin pour la dépigmentation de la peau

Cet exemple ne fait pas partie de l'invention mais peut être utile à sa compréhension.

| *Phase huileuse :* | |
|---|---|
| Triceteareth-4 phosphate/sodium C₁₄-C₁₇ alkyl sec sulfonate (Hostacerin CG vendu par la société Hoescht Celanese) (tensioactif) | 4 % |
| Alcool cetylique | 1 % |
| Vaseline | 2 % |
| Huile minérale | 4 % |
| Diméthicone | 3 % |
| Cyclométhicone | 3 % |
| Diméthicone copolyol (tensioactif) | 1 % |
| Triclosan (conservateur) | 0,1 % |
| Palmitate d'ascorbyle | 1 % |

| *Phase aqueuse* | |
|---|---|
| Propylène glycol (hydratant) | 2 % |
| PEG-20 (organoleptique) | 1 % |
| Lipolase 100 L | 1 % |
| Phénoxyéthanol (conservateur) | 0,4 % |
| Eau | qsp 100 % |

La lipolase 100 L est introduite dans la phase aqueuse sous forme encapsulée dans des microcapsules contenant aussi dé l'atélocollagène et des glycosaminoglycannes.

Ces microcapsules sont immergées dans le reste des constituants après préparation de l'émulsion.

## Revendications

1. Produit pour application topique, **caractérisé en ce qu'**il comprend au moins une enzyme qui est une lipase, au moins un précurseur d'une vitamine utilisée en cosmétique et/ou dermatologie qui est un ester comportant au moins une fonction ester à chaîne linéaire ou ramifiée, saturée ou insaturée, ayant de 2 à 25 atomes de carbone et au moins un alcool de C6 à C22, le rapport pondéral entre ledit alcool et ledit précurseur étant de 5 à 15/1.

2. Produit selon la revendication 1, **caractérisé en ce que** la chaîne de la fonction ester est choisie parmi les radicaux acyle, benzoyle, alkylbenzoyle, acylbenzoyle, 2-hydroxyphénylacétyle, éventuellement substitués.

3. Produit selon l'une des revendications précédentes, **caractérisé en ce que** la chaîne de la fonction ester a de 12 à 18 atomes de carbone.

4. Produit selon l'une quelconque des revendications précédentes, **caractérisé en ce que** l'ester est choisi parmi les esters d'acide laurique, d'acide palmitique, d'acide stéarique, d'acide cétylique, d'acide myristique, d'acide propionique, d'acide linoléique, d'acide acétique, d'acide butyrique, d'acide octanoïque, d'acide oléique, ou leurs mélanges.

5. Produit selon l'une quelconque des revendications précédentes, **caractérisé en ce que** la vitamine utilisée en cosmétique et/ou dermatologie est choisie dans le groupe comprenant le rétinol, l'acide ascorbique et le tocophérol.

6. Produit selon l'une quelconque des revendications précédentes, **caractérisé en ce que** l'ester est choisi dans le groupe comprenant le mono- et dipalmitate de dihydroxyacétone, le palmitate d'ascorbyle, le laurate d'ascorbyle, le myristate d'ascorbyle, le stéarate d'ascorbyle, le nicotinate d'ascorbyle, l'acétate de tocophérol, le linoléate de tocophérol, le palmitate de rétinyle, le propionate de rétinyle, l'acétate de rétinyle, le butyrate de rétinyle, l'octanoate de rétinyle, le laurate de rétinyle, l'oléate de rétinyle, le linoléate de rétinyle.

7. Produit selon l'une quelconque des revendications précédentes, **caractérisé en ce que** l'enzyme et le précurseur sont conditionnés de sorte à ne pas être en contact l'un avec l'autre.

8. Produit selon la revendication 7, **caractérisé en ce que** l'enzyme et le précurseur sont conditionnés dans des compartiments séparés.

9. Produit selon l'une quelconque des revendications précédentes, **caractérisé en ce que** l'enzyme et/ou le précurseur et/ou les alcools de C6 à C22 sont sous forme encapsulée.

10. Produit selon l'une quelconque des revendications précédentes, **caractérisé en ce que** l'enzyme et/ou le précurseur et/ou les alcools de C6 à C22 sont sous forme de microcapsules ou de microgranulés.

11. Produit selon l'une quelconque des revendications précédentes, **caractérisé en ce que** la lipase est choisie dans le groupe des enzymes de classification EC 3.1.1.3.

12. Produit selon l'une quelconque des revendications précédentes, **caractérisé en ce que** l'enzyme est présente en une quantité allant de 0,05 à 30 % en poids par rapport au poids total du produit.

13. Produit selon l'une quelconque des revendications précédentes, **caractérisé en ce que** l'enzyme est présente en une quantité allant de 0,1 à 10 % en poids par rapport au poids total du produit.

14. Produit selon l'une quelconque des revendications précédentes, **caractérisé en ce que** le précurseur est présent en une quantité allant de 0,1 à 50 % en poids par rapport au poids total du produit.

15. Produit selon l'une quelconque des revendications précédentes, **caractérisé en ce que**, le précurseur est présent en une quantité allant de 0,5 à 10 % en poids par rapport au poids total du produit.

16. Produit selon l'une quelconque des revendications précédentes, **caractérisé en ce que** les alcools de C6 à C22 sont des alcools dont la chaîne carbonée est saturée ou insaturée, linéaire, branché ou cyclique.

17. Produit selon l'une quelconque des revendications précédentes, **caractérisé en ce que** les alcools de C6 à C22 sont choisis parmi l'alcool cétylique, stéarylique, isostéarylique, cétylstéarylique et le béhénylstéaryloctyldodécanol.

18. Utilisation du produit selon l'une quelconque des revendications précédentes pour préparer une pommade ou un onguent dermatologique destiné au traitement thérapeutique de la peau.

19. Utilisation du produit selon l'une quelconque des revendications 1 à 17 pour le traitement cosmétique de la peau.

## Patentansprüche

1. Produkt zur topischen Anwendung, **dadurch gekennzeichnet, daß** es ein Enzym, bei dem es sich um eine Lipase handelt, mindestens einen Precursor eines in der Kosmetik und/oder Dermatologie verwendeten Vitamins, bei dem es sich um einen Ester handelt, der mindestens eine Estergruppe mit gerader oder verzweigter, gesättigter oder ungesättigter Kette mit 2 bis 25 Kohlenstoffatomen aufweist, und mindestens einen Alkohol mit 6 bis 22 Kohlenstoffatomen enthält, wobei das Gewichtsverhältnis des Alkohols und des Precursors im Bereich von 5 bis 15/1 liegt.

2. Produkt nach Anspruch 1, **dadurch gekennzeichnet, daß** die Kette der Estergruppe unter den Gruppen Acyl, Benzoyl, Alkylbenzoyl, Acylbenzoyl und 2-Hydroxyphenylacetyl ausgewählt ist, die gegebenenfalls substituiert sind.

3. Produkt nach einem der vorhergehenden Ansprüche, **dadurch gekennzeichnet, daß** die Kette der Estergruppe 12 bis 18 Kohlenstoffatome aufweist.

4. Produkt nach einem der vorhergehenden Ansprüche, **dadurch gekennzeichnet, daß** der Ester unter den Estern von Laurinsäure, Palmitinsäure, Stearinsäure, Cetylsäure, Myristinsäure, Propionsäure, Linolsäure, Essigsäure, Buttersäure, Octansäure, Ölsäure oder deren Gemischen ausgewählt ist.

5. Produkt nach einem der vorhergehenden Ansprüche, **dadurch gekennzeichnet, daß** das in der Kosmetik und/oder Dermatologie verwendete Vitamin unter Retinol, Ascorbinsäure und Tocopherol ausgewählt ist.

6. Produkt nach einem der vorhergehenden Ansprüche, **dadurch gekennzeichnet, daß** der Ester unter Dihydroxyacetonmonopalmitat, Dihydroxyacetondipalmitat, Ascorbylpalmitat, Ascorbyllaurat, Ascorbylmyristat, Ascorbylstearat, Ascorbylnicotinat, Töcopherolacetat, Tocopherollinoleat, Retinylpalmitat, Retinylpropionat, Retinylacetat, Retinylbutyrat, Retinyloctanoat, Retinyllaurat, Retinyloleat und Retinyllinoleat ausgewählt ist.

7. Produkt nach einem der vorhergehenden Ansprüche, **dadurch gekennzeichnet, daß** das Enzym und der Precursor so konfektioniert sind, daß sie nicht miteinander in Kontakt stehen.

8. Produkt nach Anspruch 7, **dadurch gekennzeichnet, daß** das Enzym und der Precursor in getrennten Abteilungen verpackt sind.

9. Produkt nach einem der vorhergehenden Ansprüche, **dadurch gekennzeichnet, daß** das Enzym und/oder der Precursor und/oder die C₆₋₂₂-Alkohole eingekapselt vorliegen.

10. Produkt nach einem der vorhergehenden Ansprüche, **dadurch gekennzeichnet, daß** das Enzym und/oder der Precursor und/oder die C₆₋₂₂-Alkohole in Form von Mikrokapseln oder Mikrogranulat vorliegen.

11. Produkt nach einem der vorhergehenden Ansprüche, **dadurch gekennzeichnet, daß** die Lipase unter den Enzymen der Klassifizierung EC 3.1.1.3 ausgewählt ist.

12. Produkt nach einem der vorhergehenden Ansprüche, **dadurch gekennzeichnet, daß** das Enzym in einem Mengenanteil von 0,05 bis 30 Gew.-%, bezogen auf das Gesamtgewicht des Produktes, vorliegt.

13. Produkt nach einem der vorhergehenden Ansprüche, **dadurch gekennzeichnet, daß** das Enzym in einem Mengenanteil von 0,1 bis 10 Gew.-%, bezogen auf das Gesamtgewicht des Produktes, vorliegt.

14. Produkt nach einem der vorhergehenden Ansprüche, **dadurch gekennzeichnet, daß** der Precursor in einem Mengenanteil von 0,1 bis 50 Gew.-%, bezogen auf das Gesamtgewicht des Produktes, vorliegt.

15. Produkt nach einem der vorhergehenden Ansprüche, **dadurch gekennzeichnet, daß** der Precursor in einem Mengenanteil von 0,5 bis 10 Gew.-%, bezogen auf das Gesamtgewicht des Produktes, vorliegt.

16. Produkt nach einem der vorhergehenden Ansprüche, **dadurch gekennzeichnet, daß** es sich bei den C₆₋₂₂-Alkoholen um Alkohole handelt, deren Kohlenstoffkette gesättigt oder ungesättigt, geradkettig, verzweigt oder cyclisch vorliegt.

17. Produkt nach einem der vorhergehenden Ansprüche, **dadurch gekennzeichnet, daß** die C₆₋₂₂-Alkohole unter Cetylalkohol, Stearylalkohol, Isostearylalkohol, Cetylstearylalkohol und Behenylstearyloctyldodecanol ausgewählt sind.

18. Verwendung eines Produktes nach einem der vorhergehenden Ansprüche zur Herstellung einer dermatologischen Pomade oder Salbe zur therapeutischen Behandlung der Haut.

19. Verwendung eines Produktes nach einem der Ansprüche 1 bis 17 zur kosmetischen Behandlung der Haut.

## Claims

1. Product for topical application, **characterized in that** it comprises at least one enzyme which is a lipase, at least one precursor of a vitamin used in cosmetics and/or dermatology which is an ester comprising at least one ester function with a linear or branched, saturated or unsaturated chain containing from 2 to 25 carbon atoms and at least one C₆ to C₂₂ alcohol, the weight ratio between the said alcohol and the said precursor being from 5 to 15/1.

2. Product according to Claim 1, **characterized in that** the chain of the ester function is chosen from acyl, benzoyl, alkylbenzoyl, acylberizoyl and 2-hydroxyphenylacetyl radicals, which are optionally substituted.

3. Product according to either of the preceding claims, **characterized in that** the chain of the ester function contains from 12 to 18 carbon atoms.

4. Product according to any one of the preceding claims, **characterized in that** the ester is chosen from lauric acid, palmitic acid, stearic acid, cetylic acid, myristic acid, propionic acid, linoleic acid, acetic acid, butyric acid, octanoic acid and oleic acid esters, or mixtures thereof.

5. Product according to any one of the preceding claims, **characterized in that** the vitamin used in cosmetics and/or dermatology is chosen from the group comprising retinol, ascorbic acid and tocopherol.

6. Product according to any one of the preceding claims, **characterized in that** the ester is chosen from the group comprising dihydroxyacetone mono-and dipalmitate, ascorbyl palmitate, ascorbyl laurate, ascorbyl myristate, ascorbyl stearate, ascorbyl nicotinate, tocopheryl acetate, tocopheryl linoleate, retinyl palmitate, retinyl propionate, retinyl acetate, retinyl butyrate, retinyl octanoate, retinyl laurate, retinyl oleate or retinyl linoleate.

7. Product according to any one of the preceding claims, **characterized in that** the enzyme and the precursor are packaged so as not to be in contact with each other.

8. Product according to Claim 7, **characterized in that** the enzyme and the precursor are packaged in separate compartments.

9. Product according to any one of the preceding claims, **characterized in that** the enzyme and/or the precursor and/or the C₆ to C₂₂ alcohols are in encapsulated form.

10. Product according to any one of the preceding claims, **characterized in that** the enzyme and/or the precursor and/or the C₆ to C₂₂ alcohols are in the form of microcapsules or microgranules.

11. Product according to any one of the preceding claims, **characterized in that** the lipase is chosen from the group of enzymes of classification EC 3.1.1.3.

12. Product according to any one of the preceding claims, **characterized in that** the enzyme is present in an amount ranging from 0.05 to 30% by weight relative to the total weight of the product.

13. Product according to any one of the preceding claims, **characterized in that** the enzyme is present in an amount ranging from 0.1 to 10% by weight relative to the total weight of the product.

14. Product according to any one of the preceding claims, **characterized in that** the precursor is present in an amount ranging from 0.1 to 50% by weight relative to the total weight of the product.

15. Product according to any one of the preceding claims, **characterized in that** the precursor is present in an amount ranging from 0.5 to 10% by weight relative to the total weight of the product.

16. Product according to any one of the preceding claims, **characterized in that** the C₆ to C₂₂ alcohols are alcohols whose carbon chain is saturated or unsaturated, and linear, branched or cyclic.

17. Product according to any one of the preceding claims, **characterized in that** the C₆ to C₂₂ alcohols are chosen from cetyl alcohol, stearyl alcohol, isostearyl alcohol, cetylstearyl alcohol and behenylstearyloctyldodecanol.

18. Use of the product according to any one of the preceding claims, to prepare a dermatological salve or ointment intended for the therapeutic treatment of the skin.

19. Use of the product according to any one of Claims 1 to 17, for the cosmetic treatment of the skin.
